(19) Europäisches Patentamt
European Patent Office
Office européen des brevets

(11) EP 4 553 039 A1

(12) **EUROPÄISCHE PATENTANMELDUNG**

(43) Veröffentlichungstag:
**14.05.2025 Patentblatt 2025/20**

(21) Anmeldenummer: **23208276.8**

(22) Anmeldetag: **07.11.2023**

(51) Internationale Patentklassifikation (IPC):
***C01B 3/36** (2006.01)* ***C01B 3/38** (2006.01)*
***C07C 29/151** (2006.01)* ***C25B 1/04** (2021.01)*

(52) Gemeinsame Patentklassifikation (CPC):
(C-Sets verfügbar)
**C01B 3/382; C01B 3/36; C01B 3/384; C07C 29/1518; C25B 1/04;** C01B 2203/0233; C01B 2203/0244; C01B 2203/025; C01B 2203/0405; C01B 2203/043; C01B 2203/061; C01B 2203/0822; C01B 2203/1241; C01B 2203/127
(Forts.)

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **L'AIR LIQUIDE, SOCIETE ANONYME POUR L'ETUDE ET L'EXPLOITATION DES PROCEDES GEORGES CLAUDE**
**75007 Paris (FR)**

(72) Erfinder:
- **Gronemann, Veronika**
  **60439 Frankfurt am Main (DE)**
- **Oelmann, Tobias**
  **60439 Frankfurt (DE)**

(74) Vertreter: **Air Liquide**
**L'Air Liquide S.A.**
**Direction de la Propriété Intellectuelle**
**75, Quai d'Orsay**
**75321 Paris Cedex 07 (FR)**

# (54) VERFAHREN ZUR BEREITSTELLUNG VON SYNTHESEGAS UND ZUR HERSTELLUNG VON METHANOL

(57) Es wird ein Verfahren zum Herstellen von Synthesegas, insbesondere von Synthesegas für die Methanolsynthese, vorgeschlagen. Das Verfahren umfasst die Schritte des Bereitstellens eines schwefelhaltigen Kohlenwasserstoffstroms; des Bereitstellens eines elektrolytisch erzeugten Wasserstoffstroms; des Zuführens eines Teils des elektrolytisch erzeugten Wasserstoffstroms zu zumindest einem Teil des schwefelhaltigen Kohlenwasserstoffstroms, wodurch ein mit Wasserstoff angereicherter schwefelhaltiger Kohlenwasserstoffstrom erhalten wird; des Entschwefelns des gemäß Schritt (c) erhaltenen Stroms in einer Hydrodesulfurierungseinheit (HDS-Einheit) (12), wodurch ein schwefelfreier Kohlenwasserstoffstrom erhalten wird; des Zuführens eines Teils des elektrolytisch erzeugten Wasserstoffstroms zu zumindest einem Teil des gemäß Schritt (d) erhaltenen Stroms, wodurch ein mit Wasserstoff angereicherter, schwefelfreier Kohlenwasserstoffstrom erhalten wird; und des Umsetzens zumindest eines Teils des gemäß Schritt (e) erhaltenen Stroms unter Beteiligung von Sauerstoff als Oxidationsmittel in einem Reformierungsschritt zu einem Synthesegasstrom.

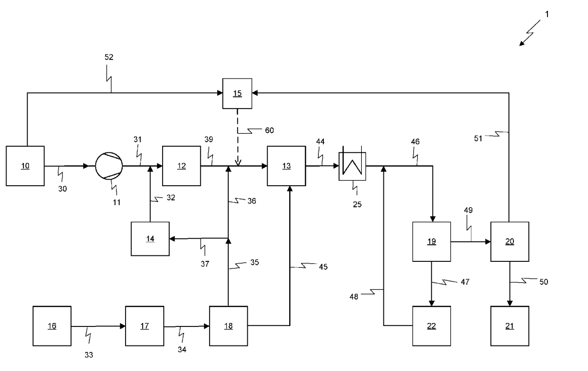


Fig. 1

EP 4 553 039 A1

(52) Gemeinsame Patentklassifikation (CPC): (Forts.)

C-Sets
**C07C 29/1518, C07C 31/04**

## Beschreibung

### Gebiet der Erfindung

Die Erfindung betrifft ein Verfahren zum Herstellen von Synthesegas. Die Erfindung betrifft ferner ein Verfahren zum Herstellen von Methanol, umfassend vorgenanntes Verfahren zum Herstellen von Synthesegas.

### Stand der Technik

Methanol wird im großtechnischen Maßstab aus Synthesegas hergestellt. Synthesegas ist ein Gemisch aus vorwiegend Wasserstoff ($H_2$), Kohlenmonoxid (CO) sowie Kohlendioxid ($CO_2$). Kohlenmonoxid sowie Kohlendioxid werden dabei häufig unter dem Begriff "Kohlenoxide" oder "Kohlenstoffoxide" zusammengefasst. Dabei laufen an einem festen Methanolsynthese-Katalysator unter anderem die beiden folgenden Gleichgewichtsreaktionen (1) und (2) nebeneinander ab.

$$(1) \quad CO_2 + 3\ H_2 \rightleftharpoons CH_3OH + H_2O$$

$$(2) \quad CO + 2\ H_2 \rightleftharpoons CH_3OH$$

Die Zusammensetzung des Synthesegases ist durch die sogenannte Stöchiometriezahl SN, definiert als

$$SN = \frac{n(H_2) - n(CO_2)}{n(CO) + n(CO_2)}\ , mit\ n\ in\ [mol],$$

charakterisiert. Eine für die Methanolsynthese stöchiometrisch ausbilanzierte Synthesegaszusammensetzung ist durch eine Stöchiometriezahl SN von 2,0 gekennzeichnet. Werte von kleiner als 2,0 weisen auf ein Wasserstoffdefizit hin, während Werte von größer als 2,0 auf einen Wasserstoffüberschuss hinweisen. Bei einem Synthesegas mit einer Stöchiometriezahl von kleiner als 2,0 wird auch von einem unterstöchiometrischen oder substöchiometrischen Synthesegas gesprochen.

Synthesegase mit Wasserstoffdefizit werden regelmäßig bei Prozessen erhalten, welche einen partiellen Oxidationsschritt umfassen. Dazu gehören partielle Oxidation (POX) als solche und autotherme Reformierung (ATR).

Die Hauptelemente eines ATR-Reaktors sind ein Brenner, eine Brennkammer und ein Katalysatorbett in einem feuerfest ausgekleideten Druckmantel. In einem ATR-Reaktor folgt auf die partielle Oxidation eines kohlenwasserstoffhaltigen Einsatzstroms durch unterstöchiometrische Mengen von Sauerstoff eine Dampfreformierung des teilweise oxidierten kohlenwasserstoffhaltigen Einsatzstroms an einem Festbett aus Dampfreformierungskatalysator.

Eine partielle Oxidation läuft für Methan als Kohlenwasserstoff vor allem nach folgender Reaktionsgleichung (3) ab.

$$(3) \quad CH_4 + 1\ ½\ O_2 \rightleftharpoons CO + 2\ H_2O$$

Die exotherme partielle Oxidation liefert somit Dampf und die erforderliche Wärmeenergie für die endotherme Dampfreformierungsreaktion, welche nach Reaktionsgleichung (4) abläuft.

$$(4) \quad CH_4 + H_2O \rightleftharpoons 3\ H_2 + CO$$

Aufgrund der hohen Temperatur findet die Dampfreformierung (4) bis zu einem gewissen Grad auch in der Brennkammer des Reaktors ohne Beteiligung des Katalysators statt.

Die Dampfreformierungsreaktion wird von der Wassergas-Shift-Reaktion gemäß Reaktionsgleichung (5) begleitet, welche ebenfalls exotherm verläuft.

$$(5) \quad CO + H_2O \rightleftharpoons CO_2 + H_2$$

Normalerweise befindet sich das Gas am Ausgang des ATR-Reaktors in Bezug auf die Dampfreformierung und die Wassergas-Shift-Reaktion im oder nahe am thermodynamischen Gleichgewicht.

Bei einer ATR wird dem eingesetzten Kohlenwasserstoff häufig Dampf zugesetzt. Die genaue Zusammensetzung des Synthesegases am Ausgang des Reaktors hängt vom Verhältnis Kohlenwasserstoff zu Dampf des Einsatzstroms, der Prozesstemperatur und dem Prozessdruck ab. Die Temperatur des Gasgemischs am Ausgang des ATR-Reaktors liegt typischerweise im Bereich von 950 bis 1050 Grad Celsius. Der Prozessdruck liegt typischerweise bei 30 bis 80 bar.

Eine Alternative zur Herstellung von Synthesegas ist die partielle Oxidation als solche, auch als POX bezeichnet. Die Hauptelemente eines POX-Reaktors sind ein Brenner und eine Brennkammer, die sich in einem feuerfest ausgekleideten Druckmantel befinden. In einem POX-Reaktor findet eine partielle Oxidation eines kohlenwasserstoffhaltigen Einsatzstroms durch unterstöchiometrische Mengen Sauerstoff statt. Es findet auch eine gewisse (nicht katalysierte) Dampfreformierung statt und die Wassergas-Shift-Reaktion begleitet die partielle Oxidation. Die obigen Reaktionsgleichungen (3) bis (5) sind somit ebenfalls anwendbar. Die Temperatur des Gasgemisches am Ausgang des Reaktors liegt typischerweise im Bereich von 1250 bis 1450 Grad Celsius. Der Prozessdruck liegt typischerweise bei 30 bis 100 bar.

Wie bereits erwähnt liefern die beiden oben beschriebenen Prozesse üblicherweise ein deutlich unterstöchiometrisches Synthesegas, also ein Synthesegas mit einer Stöchiometriezahl von deutlich kleiner als 2,0. Ein solches Synthesegas weist in Bezug auf die Synthese von Methanol ein Wasserstoff-Defizit auf. Wird ein solches Synthesegas direkt für die Herstellung von Methanol verwendet, so wird der Wasserstoff somit nahezu vollständig verbraucht, während ein wesentlicher Teil der Kohlenstoffoxide nicht umgesetzt wird. Dies hat unter anderem zur Folge, dass der Gehalt an Nebenprodukten (insbesondere höhere Alkohole und Ketone) höher ist als erwünscht und dass die Ausbeute an Methanol nicht optimal ist.

Aus diesem Grund wird in einem industriellen Prozess dem Strom des unterstöchiometrischen Synthesegases in der Regel ein Strom reinen Wasserstoffs zugeführt, um die Stöchiometriezahl SN des Synthesegases auf zumindest 1,9 zu erhöhen, vorzugsweise auf 2,0 oder mehr zu erhöhen.

Dafür wird in konventionellen Verfahren ein Teil des reformierten Synthesegases vom Hauptstrom abgetrennt und Wasserstoff aus diesem Teilstrom in der Regel durch Druckwechseladsorption *(pressure swing adsorption, PSA)* oder ein Membransystem abgetrennt. Das bei der Druckwechseladsorption anfallende Abgas oder *tail gas* enthält Kohlenwasserstoffe und/oder Kohlenmonoxid und kann daher als Brenngas für den ATR- oder POX-Reaktor verwertet werden.

Wird der Wasserstoff stattdessen durch einen Elektrolyseur bereitgestellt, kann ein Teil des für die Methanolsynthese erforderlichen Synthesegases eingespart werden, wodurch sich der $CO_2$-Fußabdruck der Anlage verbessert.

Die EP 3 658 494 B1 schlägt daher vor, Wasserstoff aus einem Elektrolyseur einem Synthesegas-Strom aus einem POX- oder ATR-Reaktor beizumischen. Zusätzlich wird Sauerstoff aus einer Luftzerlegungseinheit als Oxidationsmittel zum Betrieb des Brenners des POX- oder ATR-Reaktors verwendet.

Der von einem Elektrolyseur bereitgestellte Wasserstoff enthält üblicherweise Reste von bis zu 1 Vol.-% Sauerstoff, oder mehr. Dies hängt mit nicht zu vermeidenden Diffusionsprozessen zusammen, welche innerhalb einer Elektrolysezelle zwischen dem Anoden- und Kathodenraum ablaufen. Unter anderem diffundiert stets eine gewisse Menge des anodisch erzeugten Sauerstoffs durch die Membran oder das Diaphragma vom Anoden- in den Kathodenraum und verunreinigt dadurch den kathodisch erzeugten Wasserstoff.

Sauerstoff stellt für Methanol-Katalysatoren ein Katalysatorgift dar und muss daher möglichst quantitativ aus dem kathodisch erzeugten Wasserstoffstrom entfernt werden, bevor dieser dem Synthesegas-Strom aus der POX- oder ATR-Einheit beigemischt wird. Dies erfolgt in einer sogenannten Desoxygenierungeinheit, kurz De-Ox Einheit. Diese umfasst eine katalytische Stufe zur quantitativen Umsetzung des verunreinigenden Sauerstoffs mit Wasserstoff des Wasserstoffstroms zu Wasser. Das katalytisch so erzeugte Wasser kann anschließend adsorptiv gebunden werden, beispielsweise an einem Molekularsieb. Dadurch wird ein sauerstoff- und wasserfreier Wasserstoffstrom erhalten.

Der wichtigste kohlenwasserstoffhaltige Einsatzstoff für die Erzeugung von Synthesegas ist Erdgas. Erdgas enthält in der Regel Schwefelverbindungen, die aufgrund ihrer Wirkung als Katalysatorgift im ATR Prozess oder einer nachgeschalteten Methanolsynthese zu entfernen sind. Die Entschwefelung findet üblicherweise in einer sogenannten Hydrodesulfurierungseinheit (HDS-Einheit) statt. Schwefelverbindungen wie Mercaptane, Sulfide, Disulfide und Thiophene werden dabei unter Zuführung von Wasserstoff bei Temperaturen von 300 bis 500 °C an einem Katalysator hydriert, wobei Schwefelwasserstoff als leicht zu entfernende Schwefelverbindung gebildet wird. Der Schwefelwasserstoff kann anschließend beispielsweise mittels einer Aminwäsche aus dem Kohlenwasserstoffgemisch entfernt werden. Üblich ist es, in etwa 3 % Wasserstoffanteil im Erdgas vor der Hydrierung einzustellen.

### Beschreibung der Erfindung

Eine Aufgabe der vorliegenden Erfindung besteht darin ein Verfahren vorzuschlagen, welches die Integration der vorgenannten Verfahren dahingehend verbessert, dass insbesondere Kohlendioxidemissionen bei der Herstellung von Synthesegas und optional bei der nachfolgenden Synthese von Methanol verringert werden.

Eine weitere Aufgabe der vorliegenden Erfindung ist darin zu sehen, den Verbrauch von Erdgas oder anderen

kohlenwasserstoffhaltigen Einsatzstoffen bei der Erzeugung von Synthesegas und optional einer nachfolgenden Methanolsynthese zu verringern.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen. Bevorzugte Ausgestaltungen von Bestandteilen einer erfindungsgemäßen Kategorie sind, soweit zutreffend, ebenso bevorzugt für gleichnamige oder entsprechende Bestandteile einer jeweils anderen erfindungsgemäßen Kategorie. Die Ausdrücke "aufweisend", "umfassend" oder "beinhaltend" etc. schließen nicht aus, dass weitere Elemente, Inhaltsstoffe etc. enthalten sein können. Der unbestimmte Artikel "ein" schließt nicht aus, dass eine Mehrzahl vorhanden sein kann.

Die vorgenannten Aufgaben werden zumindest teilweise gelöst durch ein Verfahren zum Herstellen von Synthesegas, insbesondere von Synthesegas für die Methanolsynthese, umfassend die Schritte:

(a) Bereitstellen eines schwefelhaltigen Kohlenwasserstoffstroms;

(b) Bereitstellen eines elektrolytisch erzeugten Wasserstoffstroms;

(c) Zuführen eines Teils des elektrolytisch erzeugten Wasserstoffstroms zu zumindest einem Teil des schwefelhaltigen Kohlenwasserstoffstroms, wodurch ein mit Wasserstoff angereicherter schwefelhaltiger Kohlenwasserstoffstrom erhalten wird;

(d) Entschwefeln des gemäß Schritt (c) erhaltenen Stroms in einer Hydrodesulfurierungseinheit (HDS-Einheit), wodurch ein schwefelfreier Kohlenwasserstoffstrom erhalten wird;

(e) Zuführen eines Teils des elektrolytisch erzeugten Wasserstoffstroms zu zumindest einem Teil des gemäß Schritt (d) erhaltenen Stroms, wodurch ein mit Wasserstoff angereicherter, schwefelfreier Kohlenwasserstoffstrom erhalten wird;

(f) Umsetzen zumindest eines Teils des gemäß Schritt (e) erhaltenen Stroms unter Beteiligung von Sauerstoff als Oxidationsmittel in einem Reformierungsschritt zu einem Synthesegasstrom.

Erfindungsgemäß wird elektrolytisch erzeugter Wasserstoff sowohl für die Entschwefelung des schwefelhaltigen Kohlenwasserstoffstroms, als auch für die Anreicherung des erzeugten Synthesegases mit Wasserstoff genutzt. Letzteres erfolgt kontraintuitiv durch Zuführung des Wasserstoffs zum entschwefelten Kohlenwasserstoffstrom, bevor dieser dem Reformierungsschritt gemäß Schritt (f) zugeführt wird, also vor anstatt nach der Erzeugung des Synthesegases.

Der elektrolytisch erzeugte Wasserstoffstrom wird mindestens zweifach genutzt, für die Entschwefelung des schwefelhaltigen Kohlenwasserstoffstroms und für die Anreicherung von Synthesegas mit Wasserstoff. Wird ein entsprechend ausgelegter Elektrolyseur verwendet, so ist es nicht erforderlich, einen Synthesegasnebenstrom aus dem Prozess alleine zur Erzeugung von Wasserstoff abzutrennen, um diesen Wasserstoff anschließend dem Synthesegashauptstrom zur Anreicherung mit Wasserstoff zuzuführen. Dadurch nimmt der Anteil an kohlenwasserstoffhaltigem Prozessgas deutlich ab.

Durch die mindestens zweifache Nutzung von elektrolytisch erzeugtem Wasserstoff kann der erforderliche Elektrolyseur weiterhin flexibler genutzt werden. Steht aufgrund der Schwankung bei der Bereitstellung von erneuerbarem Strom weniger elektrolytisch erzeugter Wasserstoff zur Verfügung, so kann diese geringfügigere Menge zum Beispiel vollständig für die Entschwefelung und nur teilweise für die Anreicherung des Synthesegases mit Wasserstoff genutzt werden. Die für die Wasserstoffanreicherung des Synthesegases fehlende Wasserstoffmenge kann dann beispielsweise durch eine Wasserstoffrückgewinnungseinheit wie eine PSA-Einheit (Wechseldruckadsorptionseinheit) zur Verfügung gestellt werden, welche üblicherweise Teil einer nachgeschalteten Methanolsynthese ist. Dort kann Wasserstoff mittels einer Wasserstoffrückgewinnungsanlage, beispielsweise einer PSA-Einheit oder einem Membransystem, aus dem Purge-Strom abgetrennt werden.

Der elektrolytisch erzeugte Wasserstoffstrom wird dem schwefelfreien Kohlenwasserstoffstrom gemäß Schritt (e) kontraintuitiv vor der Umsetzung des Kohlenwasserstoffstroms zu Synthesegas gemäß Schritt (f) zugeführt. Der elektrolytisch erzeugte Wasserstoffstrom wird dem schwefelfreien Kohlenwasserstoffstrom insbesondere stromaufwärts des jeweiligen für den Reformierungsschritt konfigurierten Reaktor zugeführt. Bei diesem Reaktor handelt es sich insbesondere um einen POX- oder einen ATR-Reaktor. Der so erhaltene mit Wasserstoff angereicherte, schwefelfreie Kohlenwasserstoffstrom wird dann gemäß Schritt (f) unter Beteiligung von Sauerstoff als Oxidationsmittel zu Synthesegas umgesetzt.

Es ist daher erfindungsgemäß möglich, einen sauerstoffhaltigen elektrolytisch erzeugten Wasserstoffstrom für

die Umsetzung gemäß Schritt (f) zu verwenden. Würde der elektrolytisch erzeugte Wasserstoffstrom dem Synthesegasstrom gemäß Stand der Technik erst nach der Umsetzung gemäß Schritt (f) zugeführt, so erforderte dies eine quantitative Entfernung von Sauerstoff aus dem elektrolytisch erzeugten Wasserstoffstrom. Wie oben beschrieben stellt Sauerstoff für Methanolsynthese-Katalysatoren und andere Katalysatorarten ein Katalysatorgift dar.

Bei dem Reformierungsschritt gemäß Schritt (f) handelt es sich zwangsläufig um einen Reformierungsschritt, bei welchem Sauerstoff im Verhältnis zu dem eingesetzten Kohlenwasserstoff in unterstöchiometrischer Menge eingesetzt wird. Wäre dies nicht der Fall, so könnte kein Synthesegasstrom aus dem wasserstoffhaltigen Einsatzgasstrom erzeugt werden. Der eingesetzte Sauerstoff wird gemäß Schritt (f) somit vorzugsweise vollständig verbraucht, so dass der erzeugte Synthesegasstrom frei von Sauerstoff ist.

Gemäß einem Beispiel wird ein Teil des elektrolytisch erzeugten Wasserstoffstroms gemäß Schritt (c) dem vollständigen schwefelhaltigen Kohlenwasserstoffstrom zugeführt.

Gemäß einem Beispiel wird ein Teil des elektrolytisch erzeugten Wasserstoffstroms gemäß Schritt (e) dem vollständigen gemäß Schritt (d) erzeugten schwefelfreien Kohlenwasserstoffstrom zugeführt.

Gemäß einem weiteren Beispiel wird der elektrolytisch erzeugte Wasserstoffstrom vollständig, aufgeteilt auf zwei Teilströme, für die Schritte (c) und (e) genutzt.

Bei dem schwefelhaltigen Kohlenwasserstoffstrom handelt es sich vorzugsweise um einen Erdgasstrom mit Methan als Hauptkomponente. Vorzugsweise weist das Methan dabei einen Anteil von mindestens 50 Vol.-% auf, vorzugsweise mindestens 75 Vol.-%, weiter bevorzugt mindestens 90 Vol.-%, weiter bevorzugt mindestens 95 Vol.-%, weiter bevorzugt mindestens 99 Vol.-%.

Der gemäß Schritt (c) erhaltene Strom weist vorzugsweise einen Wasserstoffanteil von 1 bis 10 Vol.-% auf, vorzugweise von 1 bis 5 Vol.-%, weiter bevorzugt von 2 bis 4 Vol.-%.

Der elektrolytisch erzeugte Wasserstoffstrom kann durch jedes dem Fachmann geläufige Elektrolyse-Verfahren erzeugt werden. Vorzugsweise handelt es sich bei dem Elektrolyse-Verfahren um eine Wasserelektrolyse. Beispiele sind alkalische Elektrolyse, Protonenaustauschermembran-Elektrolyse (PEM-Elektrolyse), Anionenaustauschermembran-Elektrolyse (AEM-Elektrolyse), Hochtemperaturelektrolyse (HTE) und Elektrolyse unter Verwendung von Festoxid-Elektrolyseurzellen (SOEC).

Der Synthesegasstrom weist Synthesegas auf. Ein Synthesegas ist ein Gasgemisch, welches zumindest ein Kohlenstoffoxid (Kohlenmonoxid oder Kohlendioxid) und Wasserstoff aufweist. Vorzugsweise weist das Synthesegas Kohlenmonoxid, Kohlendioxid und Wasserstoff auf.

Unter einem Reformierungsschritt wird grundsätzlich die chemische Umsetzung von Kohlenwasserstoffen mit Sauerstoff und/oder Dampf zu einem Synthesegas verstanden.

Eine bevorzugte Ausführungsform des erfindungsgemäßen Verfahrens ist dadurch gekennzeichnet, dass der elektrolytisch erzeugte Wasserstoffstrom als Begleitsubstanz Sauerstoff enthält, und wobei

- aus demjenigen Teil des elektrolytisch erzeugten Wasserstoffstroms, welcher gemäß Schritt (e) dem schwefelfreien Kohlenwasserstoffstrom zugeführt wird, kein Sauerstoff entfernt wird, und
- aus demjenigen Teil des elektrolytisch erzeugten Wasserstoffstroms, welcher gemäß Schritt (c) dem schwefelhaltigen Kohlenwasserstoffstrom zugeführt wird, der Sauerstoff entfernt wird.

Wie weiter oben ausgeführt muss der für die Reformierung mit Sauerstoff vorgesehene, mit Elektrolysewasserstoff angereicherte Kohlenwasserstoffstrom nicht frei von Sauerstoff sein. Ein Teil des elektrolytisch erzeugten Wasserstoffstroms kann daher als Rohwasserstoffstrom im Sinne von Schritt (e) dem schwefelfreien Kohlenwasserstoffstrom zugeführt werden. Die Entfernung von Wasser aus diesem Wasserstoffteilstrom ist ebenfalls nicht erforderlich, da insbesondere in einem ATR Prozess gemäß obiger Reaktionsgleichungen sowohl Dampf gebildet, als auch verbraucht wird.

Eine bevorzugte Ausführungsform des erfindungsgemäßen Verfahrens ist dadurch gekennzeichnet, dass der Reformierungsschritt ein autothermes Reformieren (ATR) oder eine partielle Oxidation (POX) des gemäß Schritt (e) erhaltenen Stroms umfasst. Ein autothermes Reformieren als Reformierungsschritt ist dabei bevorzugt.

Eine bevorzugte Ausführungsform des erfindungsgemäßen Verfahrens ist dadurch gekennzeichnet, dass

- ein Teil des gemäß Schritt (d) erhaltenen Stroms in einem endothermen Dampfreformierungsschritt zu einem Synthesegasteilstrom umgesetzt wird, und
- ein Teil des gemäß Schritt (d) erhaltenen Stroms gemäß Schritt (e) mit Wasserstoff angereichert wird, wodurch ein mit Wasserstoff angereicherter, schwefelfreier Kohlenwasserstoffteilstrom erhalten wird, und
- die vorgenannten Teilströme zusammengeführt und gemäß Schritt (f) unter Beteiligung von Sauerstoff als Oxidationsmittel in einem Reformierungsschritt zu dem Synthesegasstrom umgesetzt werden.

Bei dem endothermen Dampfreformierungsschritt handelt es sich vorzugsweise um einen SMR *(steam methane reforming)* Prozess. Der Reformierungsschritt unter Beteiligung von Sauerstoff ist vorzugsweise ein ATR Prozess.

Gemäß der vorgenannten Ausführungsform wird ein Teil des schwefelfreien Kohlenwasserstoffstroms ohne Anreicherung mit Wasserstoff einem endothermen Dampfreformierungsschritt unterzogen. Das so erzeugte Synthesegas wird mit einem mit Wasserstoff angereichertem, schwefelfreien Kohlenwasserstoffstrom zusammengeführt. Der so erhaltene Mischstrom, welcher bereits Synthesegas aus dem endothermen Dampfreformierungsprozess enthält, wird unter Beteiligung von Sauerstoff als Oxidationsmittel dem Reformierungsschritt gemäß Schritt (f) unterzogen.

Bei dem Reformierungsschritt handelt es sich in diesem Zusammenhang insbesondere um einen autothermen Reformierungsschritt. Diesem autothermen Reformierungsschritt ist ein endothermer Dampfreformierungsschritt vorgeschaltet, in welchem ein Teil des schwefelfreien Kohlenwasserstoffstroms mit Dampf reformiert wird. Durch dieses Vorgehen wird grundsätzlich ein Synthesegas mit einer höheren Stöchiometriezahl als bei einem reinen ATR-Prozess erhalten. Aus diesem Grund ist eine geringere Menge an Wasserstoff erforderlich, um die Stöchiometriezahl des Synthesegases auf einen für die Methanolsynthese geeigneten Wert einzustellen. Entsprechend kann der Elektrolyseur kleiner dimensioniert werden.

Alternativ kann überschüssiger, elektrolytisch erzeugter Wasserstoff für die Unterfeuerung des endothermen Dampfreformierungsprozesses genutzt werden.

Eine bevorzugte Ausführungsform des Verfahrens ist daher dadurch gekennzeichnet, dass ein Teil des gemäß Schritt (b) bereitgestellten, elektrolytisch erzeugten Wasserstoffstroms als Brennstoff in dem endothermen Dampfreformierungsschritt genutzt wird.

In diesem Zusammenhang ist bevorzugt, dass der elektrolytisch erzeugte Wasserstoffstrom als Begleitsubstanz Sauerstoff enthält, und dass aus demjenigen Teil des elektrolytisch erzeugten Wasserstoffstroms, welcher als Brennstoff in dem endothermen Dampfreformierungsschritt genutzt wird, kein Sauerstoff entfernt wird.

Die Entfernung von Sauerstoff aus einem für die Unterfeuerung eines Dampfreformers genutzten Elektrolysewasserstoffteilstroms ist nicht erforderlich, da die Verbrennung die Zufuhr von Verbrennungsluft oder Sauerstoff erfordert.

Eine bevorzugte Ausführungsform des Verfahrens ist in diesem Zusammenhang dadurch gekennzeichnet, dass der als Brennstoff genutzte Teil des elektrolytisch erzeugten Wasserstoffstroms mit einem Teil des gemäß Schritt (a) bereitgestellten, schwefelhaltigen Kohlenwasserstoffstrom zusammengeführt wird, wodurch ein wasserstoff- und kohlenwasserstoffhaltiger Brennstoffmischstrom erhalten wird, welcher als Brennstoff in dem endothermen Dampfreformierungsschritt genutzt wird. Alternativ kann auch ein Teil des entschwefelten Kohlenwasserstoffstroms für den Brennstoffmischstrom genutzt werden. In diesem Fall ist keine Entschwefelung der durch den endothermen Dampfreformierungsprozess entstandenen Rauchgase (Entfernung von Schwefeloxiden) erforderlich.

Eine bevorzugte Ausführungsform des erfindungsgemäßen Verfahrens ist dadurch gekennzeichnet, dass eine gemäß Schritt (e) zum schwefelfreien Kohlenwasserstoffstrom zugeführte Menge an elektrolytisch erzeugtem Wasserstoff so eingestellt wird, dass ein gemäß Schritt (f) erhaltener Synthesegasstrom eine Stöchiometriezahl SN von 1,9 bis 2,5 aufweist, vorzugsweise von 2,0 bis 2,4 aufweist, wobei gilt

$$SN = \frac{n(H_2) - n(CO_2)}{n(CO) + n(CO_2)}\ , mit\ n\ in\ [mol].$$

Dadurch wird ein Synthesegasstrom erhalten welcher direkt, also unmittelbar, für eine nachgeschaltete Methanolsynthese verwendet werden kann. Zumindest kann der so erhaltene Synthesegasstrom für eine nachgeschaltete Methanolsynthese verwendet werden, ohne dass eine weitere Zuführung von Wasserstoff zu dem Synthesegasstrom erforderlich ist. Der so erhaltene Synthesegasstrom ist ebenso für andere Synthesen unmittelbar oder zumindest ohne weitere Zuführung von Wasserstoff einsetzbar, welche Synthesegaszusammensetzungen mit entsprechender Stöchiometriezahl erfordern.

Eine bevorzugte Ausführungsform des erfindungsgemäßen Verfahrens ist dadurch gekennzeichnet, dass der elektrolytisch erzeugte Wasserstoffstrom als Begleitsubstanz bis zu 5 Vol.-% Sauerstoff enthält, oder 0,01 bis 5 Vol.-% Sauerstoff enthält, oder 0,1 bis 3 Vol.-% Sauerstoff enthält, oder 0,1 bis 1 Vol.-% Sauerstoff enthält.

Eine bevorzugte Ausführungsform des erfindungsgemäßen Verfahrens ist dadurch gekennzeichnet, dass das Verfahren das Bereitstellen eines elektrolytisch erzeugten Sauerstoffstroms umfasst, wobei der elektrolytisch erzeugte Sauerstoffstrom als Oxidationsmittel in Schritt (f) verwendet wird.

Grundsätzlich verläuft der Reformierungsschritt gemäß Schritt (f) unter Beteiligung von Sauerstoff als Oxidationsmittel. Dabei kann Luft, mit Sauerstoff angereicherte Luft, oder reiner Sauerstoff zum Einsatz kommen. Insbesondere wird das Oxidationsmittel dem Brenner einer POX- oder ATR-Einheit zugeführt und der mit Wasserstoff angereicherte Kohlenwasserstoffstrom dort mit einer unterstöchiometrischen Menge Sauerstoff zu Synthesegas umgesetzt.

Ein Elektrolyseur erzeugt üblicherweise Sauerstoff als "Nebenprodukt", wobei dieses Nebenprodukt häufig nicht verwertet wird. Die Verwendung des elektrolytisch erzeugten Sauerstoffs als Oxidationsmittel für den Reformierungsschritt gemäß Schritt (f) stellt somit eine Verbesserung der Prozessintegration des erfindungsgemäßen Verfahrens dar.

Eine weitere Ausführungsform des erfindungsgemäßen Verfahrens ist dadurch gekennzeichnet, dass das Verfahren das Bereitstellen eines durch Luftzerlegung erzeugten Sauerstoffstroms umfasst, wobei der durch Luftzerlegung erzeugte Sauerstoffstrom als Oxidationsmittel in Schritt (f) verwendet wird.

Alternativ oder zusätzlich zur Verwendung von Elektrolyse-Sauerstoff kann das Verfahren das Bereitstellen eines durch Luftzerlegung erzeugten Sauerstoffstroms beinhalten, wobei der durch Luftzerlegung erzeugte Sauerstoffstrom als Oxidationsmittel in Schritt (f) verwendet wird.

Alternativ oder zusätzlich dazu kann der Sauerstoff dem Prozess auch über eine pipeline zur Verfügung gestellt werden.

Eine bevorzugte Ausführungsform des erfindungsgemäßen Verfahrens ist dadurch gekennzeichnet, dass das Umsetzen zumindest eines Teils des gemäß Schritt (e) erhaltenen Stroms unter Beteiligung von Sauerstoff als Oxidationsmittel in einem Reformierungsschritt zu einem Synthesegasstrom gemäß Schrift (f) unter zusätzlicher Zufuhr von Dampf erfolgt.

Optional wird dem schwefelfreien Kohlenwasserstoffstrom vor der Umsetzung gemäß Schritt (f) ein Dampfstrom zugeführt, insbesondere wenn der Reformierungsschritt ein autothermes Reformieren (ATR) umfasst.

Zusätzlicher Dampf wird vorzugsweise zugeführt, wenn der Reformierungsschritt ein autothermes Reformieren umfasst. Dadurch werden in vorteilhafter Weise Rußablagerungen auf dem Katalysator verhindert, welcher für die endotherme Dampfreformierung im ATR-Reaktor genutzt wird. Unter "zusätzlichem Dampf" wird dabei Dampf verstanden, welcher nicht durch die partielle Oxidationsreaktion oder die Reaktion von Wasserstoff zu Wasser reaktionsintern erzeugt wird. Entsprechend resultiert bei Zugabe eines Dampfstroms, abhängig davon ob die Zuführung des Dampfstroms vor oder nach der Zuführung des elektrolytisch erzeugten Wasserstoffstroms zum kohlenwasserstoffhaltigen Einsatzgasstroms erfolgt, ein dampfhaltiger und wasserstoffangereicherter Kohlenwasserstoffstrom.

Eine bevorzugte Ausführungsform des erfindungsgemäßen Verfahrens ist dadurch gekennzeichnet, dass der elektrolytisch erzeugte Wasserstoffstrom teilweise dem gemäß Schritt (f) erzeugten Synthesegasstrom zugeführt wird.

Vorzugsweise wird aus diesem Teil des elektrolytisch erzeugten Wasserstoffstroms der Sauerstoff entfernt, wenn der so erzeugte Synthesegasstrom einer nachfolgenden Methanolsynthese unterzogen wird. Besonders bevorzugt wird aus einem Teilstrom des elektrolytisch erzeugten Wasserstoffstroms der Sauerstoff entfernt, und dieser sauerstofffreie Wasserstoffteilstrom wird anschließend auf zwei weitere Wasserstoffteilströme aufgeteilt. Der erste dieser Teilströme wird gemäß Schritt (c) zumindest einem Teil des schwefelhaltigen Kohlenwasserstoffstrom zugeführt. Der zweite dieser Teilströme wird gemäß vorgenannter Ausführungsform dem gemäß Schritt (f) erzeugten Synthesegasstrom zugeführt. Vorzugsweise wird somit der mit sauerstofffreiem Wasserstoff angereicherte Synthesegasstrom einer nachfolgenden Synthese unterzogen, vorzugsweise einer Methanolsynthese unterzogen.

Die vorgenannten Aufgaben werden ferner zumindest teilweise gelöst durch ein Verfahren zum Herstellen von Methanol, umfassend das Verfahren zum Herstellen von Synthesegas nach einer der vorgenannten Ausführungsformen, weiter umfassend den Schritt des Umsetzens des Synthesegasstroms an einem festen Methanolsynthese-Katalysator zu Rohmethanol, wobei das Rohmethanol zumindest Methanol (CHsOH) und Wasser umfasst.

Vorzugsweise wird der hergestellte Synthesegasstrom unmittelbar, insbesondere ohne weiteren Modifikationsschritt, insbesondere ohne weitere Zuführung eines Wasserstoffstroms, an einem festen Methanolsynthese-Reaktor zu Rohmethanol umgesetzt. Vorzugsweise weist das für die Methanolsynthese eingesetzte Synthesegas des Synthesegasstroms eine Stöchiometriezahl SN von 1,9 bis 2,5 auf, vorzugsweise von 2,0 bis 2,4 auf.

Das für die Methanolsynthese eingesetzte und erfindungsgemäß hergestellte Synthesegas des Synthesegasstroms kann auch als Synthesefrischgas bezeichnet werden. Dieses Synthesegas ist bezüglich seiner Zusammensetzung in den meisten Fällen von demjenigen Synthesegas zu unterscheiden, welches tatsächlich in den jeweiligen Methanolsynthese-Reaktor eingeschleust wird. Wie dem Fachmann bekannt, ist die Methanolsynthese im großtechnischen Maßstab in der Regel als Syntheseschleife ausgelegt, das heißt im Methanolsynthese-Reaktor nicht umgesetztes Synthesegas wird nach Abtrennung vom auskondensierten Rohmethanol zum Reaktoreingang zurückgeführt. Dieses zurückgeführte Synthesegas, auch als Recyclegas oder Kreislaufgas bezeichnet, wird mit dem Synthesefrischgas gemischt. Das resultierende gemischte Synthesegas, wie am Reaktoreingang zusammengesetzt, kann eine Stöchiometriezahl aufweisen, welche von dem oben genannten Intervall für das Synthesefrischgas abweicht.

Eine bevorzugte Ausführungsform des erfindungsgemäßen Verfahrens zur Herstellung von Methanol ist dadurch gekennzeichnet, dass das Rohmethanol in einem thermischen Trennverfahren in Reinmethanol und Wasser aufgetrennt wird, und wobei in dem thermischen Trennverfahren ein kohlenstoffhaltiger Abgasstrom erhalten wird, wobei der kohlenstoffhaltige Abgasstrom als Brennstoff in einer Heizvorrichtung zum Vorheizen des gemäß Schritt (e) erhaltenen Stroms genutzt wird, bevor dieser Strom gemäß Schritt (f) zu Synthesegas umgesetzt wird.

Im Zuge der Destillation von Rohmethanol zur Gewinnung von Reinmethanol fällt üblicherweise ein Strom von leichtsiedenden Nebenprodukten - hier als Abgasstrom bezeichnet - an, welcher als Brennstoff in einer Heizvorrichtung

zum Vorheizen es gemäß Schritt (e) erhaltenen Stroms genutzt werden kann. Vorzugsweise wird der gemäß Schritt (e) erhaltene Strom optional zusammen mit Dampf vorgeheizt, bevor dieser dem eigentlichen Reformierungsschritt gemäß Schritt (f) unterzogen wird. Bei der Heizvorrichtung handelt es sich insbesondere um eine befeuerte Heizvorrichtung.

Durch die vorgenannte Nutzung des bei der Destillation von Rohmethanol anfallenden Abgasstroms wird die Prozessintegration weiter verbessert.

Eine bevorzugte Ausführungsform des erfindungsgemäßen Verfahrens zur Herstellung von Methanol ist dadurch gekennzeichnet, dass das im thermischen Trennverfahren abgetrennte Wasser als Ausgangsmaterial für den elektrolytisch erzeugten Wasserstoffstrom verwendet wird.

Vorzugsweise wird das im thermischen Trennverfahren abgetrennte Wasser aufgearbeitet, und anschließend als Ausgangsmaterial für den elektrolytisch erzeugten Wasserstoff verwendet. Häufig enthält das im thermischen Trennverfahren abgetrennte Wasser Natriumhydroxid (NaOH), welches beispielsweise für die Nutzung des Wassers in einer PEM-Elektrolyse entfernt werden muss. Wird der elektrolytisch erzeugte Wasserstoffstrom durch eine alkalische Elektrolyse erzeugt, ist die Entfernung von Natriumhydroxid nicht zwingend erforderlich, da für die alkalische Elektrolyse hochkonzentrierte Kalilauge ($KOH_{aq}$) oder Natronlauge ($NaOH_{aq}$) als Elektrolysemedium verwendet wird.

Durch die Nutzung des im thermischen Trennverfahren abgetrennten Wassers als Ausgangsmaterial für den elektrolytisch erzeugten Wasserstoff wird die Prozessintegration verbessert, da weniger Ressourcen für die Bereitstellung von Wasser für die Wasserelektrolyse erforderlich sind.

Eine bevorzugte Ausführungsform des erfindungsgemäßen Verfahrens zur Herstellung von Methanol ist dadurch gekennzeichnet, dass beim Umsetzen des Synthesegasstroms an dem festen Methanolsynthese-Katalysator zu Rohmethanol ein Restgasstrom erzeugt wird, welcher nicht zu Rohmethanol umgesetztes Synthesegas enthält, und wobei von dem Restgasstrom ein Teil als Spülgasstrom abgetrennt wird, und wobei der Spülgasstrom einer Wasserstoffrückgewinnungsvorrichtung zugeführt wird, wodurch ein nicht elektrolytisch erzeugter Wasserstoffstrom erzeugt wird, und

- der nicht elektrolytisch erzeugte Wasserstoffstrom zumindest teilweise zusätzlich dem schwefelhaltigen Kohlenwasserstoffstrom zugeführt wird, wodurch der mit Wasserstoff angereicherte schwefelhaltige Kohlenwasserstoffstrom erhalten wird, und/oder

- der nicht elektrolytisch erzeugte Wasserstoffstrom zumindest teilweise zusätzlich dem gemäß Schritt (d) erhaltenen Strom zugeführt wird, wodurch der mit Wasserstoff angereicherte, schwefelfreie Kohlenwasserstoffstrom erhalten wird.

Die Synthese von Methanol erfolgt im industriellen Maßstab wie vorgehend beschreiben üblicherweise innerhalb einer sogenannten Syntheseschleife. Die Umsetzung des Synthesegases am Methanolsynthese-Katalysator ist aufgrund der Einstellung eines thermodynamischen Gleichgewichts nicht vollständig, und am Ausgang des jeweiligen Reaktors wird neben kondensierbarem Rohmethanol auch ein nicht kondensierbarer Restgasstrom erhalten, welcher nicht umgesetztes Synthesegas enthält. Von diesem Restgasstrom wird ein Teil zum Reaktoreingang zur erneuten Umsetzung zu Rohmethanol zurückgeführt. Dieser zurückgeführte Strom wird als Rückführgasstrom, Recyclegasstrom oder Kreislaufgasstrom bezeichnet. Ein Teil wird als Spülgasstrom abgetrennt, um die Akkumulation von unter den Bedingungen der Methanolsynthese inerten Verbindungen in der Syntheseschleife zu vermeiden.

In vorteilhafter Weise wird dieser Spülgasstrom, welcher Wasserstoff aus dem nicht umgesetzten Synthesegas (Restgasstrom) enthält, einer Wasserstoffrückgewinnungsvorrichtung zugeführt, um damit einen nicht elektrolytisch erzeugten Wasserstoffstrom zu erzeugen. Bei der Wasserstoffrückgewinnungsvorrichtung handelt es sich vorzugsweise um eine Druckwechseladsorptionsvorrichtung (PSA). Alternativ kann es sich auch um eine Membraneinheit handeln.

Der nicht elektrolytisch erzeugte Wasserstoffstrom kann zumindest teilweise zusätzlich dem schwefelhaltigen Kohlenwasserstoffstrom zugeführt wird, wodurch der mit Wasserstoff angereicherte schwefelhaltige Kohlenwasserstoffstrom erhalten wird. Zusätzlich oder alternativ kann der nicht elektrolytisch erzeugte Wasserstoffstrom zumindest teilweise zusätzlich dem gemäß Schritt (d) erhaltenen Strom zugeführt wird, wodurch der mit Wasserstoff angereicherte, schwefelfreie Kohlenwasserstoffstrom erhalten wird.

Der nicht elektrolytisch erzeugte Wasserstoffstrom kann somit den elektrolytisch erzeugten Wasserstoffstrom ergänzen, entweder bei der Erzeugung des mit Wasserstoff angereicherten schwefelhaltigen Kohlenwasserstoffstroms und/oder bei der Erzeugung des mit Wasserstoff angereicherten schwefelfreien Kohlenwasserstoffstroms. In letzterem Fall wird zusammen mit dem elektrolytisch erzeugten Wasserstoffstrom so viel nicht elektrolytisch erzeugter Wasserstoff zugeführt, dass durch Schritt (f) wiederum vorzugsweise ein Synthesegas mit einer Stöchiometriezahl SN von 1,9 bis 2,5 erhalten wird, vorzugsweise mit einer Stöchiometriezahl SN von 2,0 bis 2,4 erhalten wird.

Alternativ oder zusätzlich kann der nicht elektrolytisch erzeugte Wasserstoffstrom dem Synthesegasstrom stromabwärts des jeweiligen Reformierungsschritts mit Sauerstoff als Oxidationsmittel (POX oder ATR) und stromaufwärts zur Methanolsynthese zugeführt werden.

Da in einer Methanolanlage mit Syntheseschleife stets ein Spülgasstrom anfällt, kann dieser in vorteilhafter Weise prozessintern genutzt werden. Dadurch können insbesondere Schwankungen in der Wasserstoffproduktion eines Elektrolyseurs ausgeglichen werden, beispielsweise wenn dieser Elektrolyseur Wasserstoff auf Basis erneuerbaren Stroms produziert, welcher zeitlich nicht konstant zur Verfügung steht.

## Ausführungsbeispiele

Die Erfindung wird im Folgenden durch Ausführungsbeispiele und ein Zahlenbeispiel näher erläutert, ohne den Gegenstand der Erfindung dadurch zu beschränken. In den Zeichnungen sind funktionell und/oder strukturell gleiche oder zumindest ähnliche Bestandteile mit gleichen Bezugszeichen versehen.

Es zeigt

Figur 1 ein Blockfließbild des erfindungsgemäßen Verfahren gemäß einem ersten Beispiel, und

Figur 2 ein Blockfließbild des erfindungsgemäßen Verfahrens gemäß einem zweiten Beispiel.

Figur 1 zeigt ein vereinfachtes Blockfließbild eines Verfahrens 1 zur Synthesegasherstellung und nachfolgenden Methanolherstellung gemäß einem ersten Beispiel der Erfindung.

Aus einer Erdgasquelle 10, welche hauptsächlich Methan als Kohlenwasserstoff enthält, wird ein schwefelhaltiger Kohlenwasserstoffstrom über eine Leitung 30 zunächst einem Verdichter 11 zugeführt und anschließend in komprimierter Form über Leitung 31 weitergeführt.

Aus einer Wasserquelle 16 wird über eine Leitung 33 Rohwasser einer Wasseraufbereitungseinheit 17 zugeführt. In der Wasseraufbereitungseinheit 17 wird das Rohwasser so aufgearbeitet, dass es für eine nachfolgende Wasserelektrolyse geeignet ist. Die Aufarbeitung umfasst beispielsweise eine Filtration, die Entfernung von gelösten Salzen, und eine Entgasung. Das aufbereitete Wasser wird über Leitung 34 einem Elektrolyseur 18 zugeführt, bei dem es sich beispielhaft um einen PEM-Elektrolyseur handeln kann. Das Elektrolysemedium ist in diesem Fall das durch die Wasseraufbereitungseinheit 17 aufbereitete Wasser.

Der Elektrolyseur 18 erzeugt einen Wasserstoffstrom und einen Sauerstoffstrom. Der Wasserstoffstrom wird über eine Leitung 35 aus dem Elektrolyseur 18 abgezogen und nachfolgend auf zwei Teilströme aufgeteilt. Der erste Teilstrom wird über eine Leitung 37 weitergeführt und einer Desoxygenierungseinheit 14 zugeführt. In der Desoxygenierungseinheit 14 wird der elektrolytisch erzeugte Wasserstoffstrom von Sauerstoff befreit. Dieser von Sauerstoff befreite Wasserstoffstrom wird über eine Leitung 32 weitergeführt und in Leitung 31 mit dem schwefelhaltigen Kohlenwasserstoffstrom gemischt, so dass in Leitung 31 ein mit Wasserstoff angereicherter Kohlenwasserstoffstrom resultiert. Dieser mit Wasserstoff angereicherte Kohlenwasserstoffstrom weist einen Wasserstoffgehalt von etwa 3 Vol.-% auf. Der mit Wasserstoff angereicherte, schwefelhaltige Kohlenwasserstoffstrom wird in eine Hydrodesulfurierungseinheit 12 (HDS-Einheit 12) weitergeführt, in welcher der Kohlenwasserstoffstrom von Schwefelverbindungen befreit wird.

Der dadurch entschwefelte Kohlenwasserstoffstrom wird über eine Leitung 39 aus der HDS-Einheit 12 ausgeleitet.

Der zweite in dem Elektrolyseur 18 erzeugte Wasserstoffteilstrom wird über eine Leitung 36 weitergeführt und keiner weiteren Aufreinigung durch eine Desoxygenierungseinheit unterzogen. Dieser Wasserstoffteilstrom wird über die Leitung 36 einer Leitung 39 zugeführt, so dass in dieser Leitung 39 ein mit Wasserstoff angereicherter, schwefelfreier Kohlenwasserstoffstrom erhalten wird. Dabei wird die zugeführte Wasserstoffmenge so eingestellt, dass in der nachfolgenden Reformierungsreaktion ein Synthesegas mit einer vorbestimmten Stöchiometriezahl SN erhalten wird.

Im Elektrolyseur 18 wird auch ein Sauerstoffstrom erzeugt, welcher über eine Leitung 45 aus dem Elektrolyseur 18 ausgeleitet wird. Dieser Sauerstoffstrom wird einem ATR-Reaktor 13 (d.h. autothermen Reformer 13) zugeführt. Dem ATR-Reaktor 13 wird auch der mit Wasserstoff angereicherte, schwefelfreie Kohlenwasserstoffstrom aus Leitung 39 zugeführt. In dem ATR-Reaktor 13 werden die vorgenannten Ströme zu einem Synthesegasstrom reformiert, welcher über eine Leitung 44 aus dem ATR-Reaktor ausgeleitet wird. Der im ATR-Reaktor 13 erzeugte Synthesegasstrom wird in einer Wärmerückgewinnungseinheit 25 abgekühlt. In der Wärmerückgewinnungseinheit 25 wird gleichzeitig Dampf erzeugt, welcher optional zur Reformierung des mit Wasserstoff angereicherten Kohlenwasserstoffstroms im ATR-Reaktor 13 genutzt werden kann (nicht gezeigt).

Der abgekühlte Synthesegasstrom wird über eine Leitung 46 aus der Wärmerückgewinnungseinheit 25 ausgeleitet. Dieser wird anschließend über die selbe Leitung 46 einer Methanolsynthese-Einheit 19 zugeführt, welche zumindest über einen Reaktor mit einem festen Methanolsynthese-Katalysator, einen Kondensator und einen Gas-Flüssigkeit-Abscheider für die Erzeugung von Rohmethanol verfügt (nicht gezeigt). In der Methanolsynthese-Einheit 19 wird nicht umgesetztes Synthesegas als Kreislaufgas zum Reaktoreingang zurückgeführt (nicht gezeigt). Aus diesem Kreislaufgasstrom wird ein Anteil als Spülgasstrom entnommen, um die Anreicherung von unter den Bedingungen der Methanolsynthese inerten Komponenten in der Methanolsynthese-Einheit 19 zu verhindern. Dieser Spülgasstrom wird

über eine Leitung 47 aus der Methanolsynthese-Einheit 19 ausgeleitet und anschließend einer Wasserstoffrückgewinnungseinheit, hier einer PSA-Einheit 22, zugeführt. In der PSA-Einheit 22 wird aus dem Spülgasstrom ein in diesem Fall nicht elektrolytisch erzeugter Wasserstoffstrom und ein Abgasstrom erzeugt. Der Wasserstoffstrom wird über eine Leitung 48 aus der PSA-Einheit ausgeleitet und dem abgekühlten Synthesegasstrom in Leitung 46 zugeführt. Der nicht elektrolytisch erzeugte Wasserstoffstrom dient damit dem Einstellen der für die Methanolsynthese gewünschten Stöchiometriezahl des der Methanolsynthese-Einheit 19 zugeführten Synthesegases. Die für die Methanolsynthese gewünschte Stöchiometriezahl könnte gleichermaßen alleine durch die Zuführung des elektrolytisch erzeugten Wasserstoffstroms aus Leitung 36 zur Leitung 39 eingestellt werden. In diesem Fall kann der in der PSA-Einheit erzeugte Wasserstoff anderweitig genutzt werden.

Das in der Methanolsynthese-Einheit 19 erzeugte Rohmethanol umfasst im Wesentlichen Methanol, Wasser und unerwünschte Nebenprodukte. Das Rohmethanol wird über eine Leitung 49 einer Destillationseinheit 20 zur thermischen Trennung in die gewünschten Komponenten zugeführt. Die Destillationseinheit 20 kann eine oder mehrere Destillationskolonnen umfassen. In der Destillationseinheit 20 wird zumindest Reinmethanol mit einer vorspezifizierten Reinheit und Wasser erzeugt. Das erzeugte Wasser kann der Wasseraufbereitungseinheit 17 zugeführt werden, um als Edukt im Elektrolyseur 18 genutzt zu werden. In der Destillationseinheit 20 wird auch ein kohlenstoffhaltiger Abgasstrom leichtsiedener Substanzen erzeugt. Dieser Abgasstrom wird über eine Leitung 51 aus der Destillationseinheit 20 ausgeleitet und einer befeuerten Heizung 15 zugeführt. Die befeuerte Heizung 15 dient der Vorwärmung des mit Wasserstoff angereicherten, schwefelfreien Kohlenwasserstoffstroms, bevor dieser dem ATR-Reaktor 13 zugeführt wird. Zu diesem Zweck erzeugt die befeuerte Heizung 15 einen Wärmestrom 60, welcher durch den gestrichelten Pfeil angezeigt ist. Der befeuerten Heizung 15 wird auch ein Teil des aus der Erdgasquelle 10 stammenden Kohlenwasserstoffstrom zugeführt. Um die Kohlendioxidemissionen der befeuerten Heizung 15 zu senken, kann dieser auch nicht im Verfahren benötigter Wasserstoff aus dem Elektrolyseur 18 oder der PSA-Einheit 22 als Brennstoff zugeführt werden.

Das in der Destillationseinheit 20 erzeugte Reinmethanol wird über eine Leitung 50 aus der Destillationseinheit 20 abgezogen und als Methanolprodukt 21 einerweiteren Verwertung unterzogen.

Figur 2 zeigt ein vereinfachtes Blockfließbild eines Verfahrens 2 zur Synthesegasherstellung und nachfolgenden Methanolherstellung gemäß einem zweiten Beispiel der Erfindung. Im Folgenden wird im Wesentlichen auf Unterschiede des Verfahrens 2 zum Verfahren 1 eingegangen.

Im Verfahren 2 gemäß Figur 2 wird aus der HDS-Einheit 12 ein schwefelfreier Kohlenwasserstoffstrom über eine Leitung 42 abgezogen. Aus dieser Leitung 42 wird ein Teilstrom des entschwefelten Kohlenwasserstoffstroms über eine Leitung 41 abgezogen. Der verbleibende Kohlenwasserstoffteilstrom wird über die Leitung 42 einer SMR-Einheit 23 zugeführt und dort einer endothermen Dampfreformierung unterzogen.

Der Elektrolyseur 18 erzeugt wie im Beispiel gemäß Figur 1 einen Wasserstoffstrom, welcher auf zwei Teilströme in einer Leitung 37 und einer Leitung 38 aufgeteilt wird. Aus der Leitung 42 wird ein Teil des schwefelfreien Kohlenwasserstoffstroms abgezogen und über eine Leitung 41 weitergeführt. Der Wasserstoffteilstrom in Leitung 38 wird diesem Kohlenwasserstoffteilstrom in Leitung 41 zugeführt, wodurch ein mit Wasserstoff angereicherter, schwefelfreier Kohlenwasserstoffteilstrom erhalten wird. Dieser wird über eine Leitung 40 weitergeführt und in Leitung 43 mit einem Synthesegasteilstrom zusammengeführt, welcher als Produkt der endothermen Dampfreformierung aus der SMR-Einheit 23 ausgeleitet wird. In Leitung 43 resultiert dadurch ein Gemisch aus Synthesegas und mit Wasserstoff angereichertem Kohlenwasserstoff. Dieses Gemisch wird einem weiteren Reformierungsschritt, diesmal unter Beteiligung von Sauerstoff, in dem ATR-Reaktor 13 unterzogen. Der Sauerstoff für die Reformierung im ATR-Reaktor 13 wird analog dem Beispiel gemäß Figur 1 durch einen durch den Elektrolyseur bereitgestellten Sauerstoffstrom geliefert, welcher über die Leitung 45 dem ATR-Reaktor zugeführt wird.

Für die SMR-Einheit 23 wird aufgrund der Endothermie der Dampfreformierungsreaktion eine SMR Befeuerung 24 benötigt, welche üblicherweise aus mehreren Reihen Brennern besteht, welche in einem dem Fachmann bekannten Reformerofen reihig zur Befeuerung von katalysatorbefüllten Reformerrohren angeordnet sind. Der für die Brenner benötigte Sauerstoff wird aus der Leitung 45 abgezweigt und über eine Leitung 54 der SMR-Befeuerung 24 zugeführt. Gleichzeitig wird dafür benötigter Brennstoff aus der Erdgasquelle 10 entnommen und der SMR Befeuerung über Leitung 53 zugeführt. Die von der SMR Befeuerung bereitgestellte thermische Energie ist als der SMR-Einheit zufließender Wärmestrom 61 in Form eines gestrichelten Pfeils dargestellt.

Durch Reformierung des durch die SMR-Einheit 23 bereitgestellten Synthesegasteilstroms und des mit Wasserstoff angereicherten, schwefelfreien Kohlenwasserstoffstroms im ATR-Reaktor 13 wird ein Synthesegasstrom erhalten, dessen Stoffgemisch eine größere Stöchiometriezahl aufweist als der gemäß dem Verfahren der Figur 1 erhältliche Synthesegasstrom. Entsprechend muss diesem Synthesegasstrom weniger Wasserstoff über Leitung 48 aus der PSA-Einheit 22 zugeführt werden, und/oder dem Kohlenwasserstoffstrom in Leitung 41 muss von vorneherein weniger Wasserstoff über Leitung 38 zugeführt werden.

Das folgende Zahlenbeispiel basiert auf Simulationsdaten und dient der weiteren Erläuterung der Erfindung.

Die folgende Tabelle zeigt Simulationsdaten zweier Vergleichsbeispiele 1 und 2, sowie eines Beispiels gemäß der Erfindung.

| | Einheit | Vergleichsbeispiel 1 | Vergleichsbeispiel 2 | Beispiel gemäß Erfindung |
|---|---|---|---|---|
| Erdgasverbrauch gesamt | kmol/h | 4360 | 4316 | 4086 |
| Erdgas zum Prozess | kmol/h | 4065 | 4065 | 3573 |
| Erdgas zur befeuerten Heizung | kmol/h | 139 | 180 | 308 |
| Erdgas zum Dampferzeuger | kmol/h | 156 | 70 | 205 |
| Produktmenge | t/h | 125 | 125 | 125 |
| Synthesegas zur PSA | kmol/h | 1980 | 1480 | 0 |
| Durch PSA erzeugter Wasserstoff | kmol/h | 1433 | 1243 | 318 |
| Elektrolytisch erzeugter Wasserstoff | kmol/h | 0 | 126 | 808 |
| Kohlendioxid-Emissionen der befeuerten Heizung | kg/h | 44663 | 42066 | 28033 |
| Kohlendioxid-Emissionen des Dampferzeugers | kg/h | 8033 | 8412 | 10571 |
| Einsparung an Erdgas | % | n/a | 1,02 | 6,28 |

Prinzipiell werden gemäß jedem der Beispiele 3000 t Methanol pro Tag produziert, wobei ein autothermer Reformer (ATR) zur Erzeugung des Synthesegases zum Einsatz kommt.

Gemäß Vergleichsbeispiel 1 wird ein Teil des reformierten Gases (Synthesegases) für die Gewinnung von Wasserstoff in einer PSA-Einheit verwendet, um so Wasserstoff für das Einstellen der Stöchiometriezahl des erzeugten Synthesegases und für die HDS-Einheit zu gewinnen. Entsprechend wird dieser Wasserstoff ausschließlich durch eine PSA erzeugt, die als Ausgangsmaterial auch Spülgas aus der Methanolsynthese erhalten kann. Im Vergleichsbeispiel 1 wird Wasserstoff somit nicht durch Elektrolyse erzeugt.

Gemäß Vergleichsbeispiel 2 wird der Großteil des benötigten Wasserstoffs weiterhin durch eine PSA-Einheit erzeugt, welcher reformiertes Gas (Synthesegas) zugeführt wird. Dieser wird für die Einstellung der Stöchiometriezahl des erzeugten Synthesegases für die Methanolsynthese verwendet. Der für die HDS-Einheit benötigte Wasserstoff wird durch einen Elektrolyseur bereitgestellt.

Gemäß dem erfindungsgemäßen Beispiel wird der Großteil des erforderlichen Wasserstoffs durch einen Elektrolyseur bereitgestellt. Die PSA-Einheit erzeugt Wasserstoff ausschließlich aus dem Spülgas der Methanolsynthese. Ihr wird kein reformiertes Gas (Synthesegas) zugeführt. Entsprechend wird der Großteil des für das Einstellen der Stöchiometriezahl des Synthesegases und für die HDS-Einheit benötigten Wasserstoffs durch den Elektrolyseur bereitgestellt. Für diese Vorgehensweise zeigen sich entsprechende Einsparungen von mehr als 5 % Erdgas gegenüber dem zweiten Vergleichsbeispiel und mehr als 6 % Erdgas gegenüber dem ersten Vergleichsbeispiel. Überraschenderweise zeigen sich auch Vorteile in Bezug auf den Kohlendioxidausstoß der verwendeten befeuerten Heizvorrichtung. Der Kohlendioxid-Mehrausstoß eines Dampferzeugers im erfindungsgemäßen Beispiel gegenüber dem Vergleichsbeispiel 2 wird dadurch deutlich überkompensiert.

**Bezugszeichenliste**

| | |
|---|---|
| 1, 2 | Verfahren |
| 10 | Erdgasquelle |
| 11 | Verdichter |
| 12 | HDS-Einheit |
| 13 | ATR-Reaktor |
| 14 | Desoxygenierungseinheit |
| 15 | Befeuerte Heizung |

| | |
|---|---|
| 16 | Wasserquelle |
| 17 | Wasseraufbereitungseinheit |
| 18 | Elektrolyseur |
| 19 | Methanolsynthese-Einheit |
| 20 | Destillationseinheit |
| 21 | Methanolprodukt |
| 22 | PSA-Einheit |
| 23 | SMR-Einheit |
| 24 | SMR Befeuerung |
| 25 | Wärmerückgewinnungseinheit |
| 30 bis 54 | Leitung |
| 60, 61 | Wärmestrom |

**Patentansprüche**

1. Verfahren (1, 2) zum Herstellen von Synthesegas, insbesondere von Synthesegas für die Methanolsynthese, umfassend die Schritte:

   (a) Bereitstellen eines schwefelhaltigen Kohlenwasserstoffstroms;
   (b) Bereitstellen eines elektrolytisch erzeugten Wasserstoffstroms;
   (c) Zuführen eines Teils des elektrolytisch erzeugten Wasserstoffstroms zu zumindest einem Teil des schwefelhaltigen Kohlenwasserstoffstroms, wodurch ein mit Wasserstoff angereicherter schwefelhaltiger Kohlenwasserstoffstrom erhalten wird;
   (d) Entschwefeln des gemäß Schritt (c) erhaltenen Stroms in einer Hydrodesulfurierungseinheit (HDS-Einheit) (12), wodurch ein schwefelfreier Kohlenwasserstoffstrom erhalten wird;
   (e) Zuführen eines Teils des elektrolytisch erzeugten Wasserstoffstroms zu zumindest einem Teil des gemäß Schritt (d) erhaltenen Stroms, wodurch ein mit Wasserstoff angereicherter, schwefelfreier Kohlenwasserstoffstrom erhalten wird;
   (f) Umsetzen zumindest eines Teils des gemäß Schritt (e) erhaltenen Stroms unter Beteiligung von Sauerstoff als Oxidationsmittel in einem Reformierungsschritt zu einem Synthesegasstrom.

2. Verfahren nach Anspruch 1, **dadurch gekennzeichnet, dass** der elektrolytisch erzeugte Wasserstoffstrom als Begleitsubstanz Sauerstoff enthält, und wobei

   - aus demjenigen Teil des elektrolytisch erzeugten Wasserstoffstroms, welcher gemäß Schritt (e) dem schwefelfreien Kohlenwasserstoffstrom zugeführt wird, kein Sauerstoff entfernt wird, und
   - aus demjenigen Teil des elektrolytisch erzeugten Wasserstoffstroms, welcher gemäß Schritt (c) dem schwefelhaltigen Kohlenwasserstoffstrom zugeführt wird, der Sauerstoff entfernt wird.

3. Verfahren nach Anspruch 1 oder 2, **dadurch gekennzeichnet, dass** der Reformierungsschritt ein autothermes Reformieren (ATR) oder eine partielle Oxidation (POX) des gemäß Schritt (e) erhaltenen Stroms umfasst.

4. Verfahren nach einem der vorherigen Ansprüche, **dadurch gekennzeichnet, dass**

   - ein Teil des gemäß Schritt (d) erhaltenen Stroms in einem endothermen Dampfreformierungsschritt zu einem Synthesegasteilstrom umgesetzt wird, und
   - ein Teil des gemäß Schritt (d) erhaltenen Stroms gemäß Schritt (e) mit Wasserstoff angereichert wird, wodurch ein mit Wasserstoff angereicherter, schwefelfreier Kohlenwasserstoffteilstrom erhalten wird, und
   - die vorgenannten Teilströme zusammengeführt und gemäß Schritt (f) unter Beteiligung von Sauerstoff als Oxidationsmittel in einem Reformierungsschritt zu dem Synthesegasstrom umgesetzt werden.

5. Verfahren nach Anspruch 4, **dadurch gekennzeichnet, dass** ein Teil des gemäß Schritt (b) bereitgestellten, elektrolytisch erzeugten Wasserstoffstroms als Brennstoff in dem endothermen Dampfreformierungsschritt genutzt wird.

6. Verfahren nach Anspruch 5, **dadurch gekennzeichnet, dass** der elektrolytisch erzeugte Wasserstoffstrom als Begleitsubstanz Sauerstoff enthält, und dass aus demjenigen Teil des elektrolytisch erzeugten Wasserstoffstroms, welcher als Brennstoff in dem endothermen Dampfreformierungsschritt genutzt wird, kein Sauerstoff entfernt wird.

7. Verfahren nach Anspruch 5 oder 6, **dadurch gekennzeichnet, dass** der als Brennstoff genutzte Teil des elektrolytisch erzeugten Wasserstoffstroms mit einem Teil des gemäß Schritt (a) bereitgestellten, schwefelhaltigen Kohlenwasserstoffstrom zusammengeführt wird, wodurch ein wasserstoff- und kohlenwasserstoffhaltiger Brennstoffmischstrom erhalten wird, welcher als Brennstoff in dem endothermen Dampfreformierungsschritt genutzt wird.

8. Verfahren nach einem der vorherigen Ansprüche, **dadurch gekennzeichnet, dass** eine gemäß Schritt (e) zum schwefelfreien Kohlenwasserstoffstrom zugeführte Menge an elektrolytisch erzeugtem Wasserstoff so eingestellt wird, dass ein gemäß Schritt (f) erhaltener Synthesegasstrom eine Stöchiometriezahl SN von 1,9 bis 2,5 aufweist, vorzugsweise von 2,0 bis 2,4 aufweist, wobei gilt

$$SN = \frac{n(H_2) - n(CO_2)}{n(CO) + n(CO_2)}, mit\ n\ in\ [mol].$$

9. Verfahren nach einem der vorherigen Ansprüche, **dadurch gekennzeichnet, dass** der elektrolytisch erzeugte Wasserstoffstrom als Begleitsubstanz bis zu 5 Vol.-% Sauerstoff enthält, oder 0,01 bis 5 Vol.-% Sauerstoff enthält, oder 0,1 bis 3 Vol.-% Sauerstoff enthält, oder 0,1 bis 1 Vol.-% Sauerstoff enthält.

10. Verfahren nach einem der vorherigen Ansprüche, **dadurch gekennzeichnet, dass** das Verfahren das Bereitstellen eines elektrolytisch erzeugten Sauerstoffstroms umfasst, wobei der elektrolytisch erzeugte Sauerstoffstrom als Oxidationsmittel in Schritt (f) verwendet wird.

11. Verfahren nach einem der vorherigen Ansprüche, **dadurch gekennzeichnet, dass** das Verfahren das Bereitstellen eines durch Luftzerlegung erzeugten Sauerstoffstroms umfasst, wobei der durch Luftzerlegung erzeugte Sauerstoffstrom als Oxidationsmittel in Schritt (f) verwendet wird.

12. Verfahren nach einem der vorherigen Ansprüche, **dadurch gekennzeichnet, dass** das Umsetzen zumindest eines Teils des gemäß Schritt (e) erhaltenen Stroms unter Beteiligung von Sauerstoff als Oxidationsmittel in einem Reformierungsschritt zu einem Synthesegasstrom gemäß Schrift (f) unter zusätzlicher Zufuhr von Dampf erfolgt.

13. Verfahren nach einem der vorherigen Ansprüche, **dadurch gekennzeichnet, dass** der elektrolytisch erzeugte Wasserstoffstrom teilweise dem gemäß Schritt (f) erzeugten Synthesegasstrom zugeführt wird.

14. Verfahren zum Herstellen von Methanol, umfassend das Verfahren zum Herstellen von Synthesegas nach einem der vorherigen Ansprüche, weiter umfassend den Schritt des Umsetzens des Synthesegasstroms an einem festen Methanolsynthese-Katalysator zu Rohmethanol, wobei das Rohmethanol zumindest Methanol (CHsOH) und Wasser umfasst.

15. Verfahren nach Anspruch 14, **dadurch gekennzeichnet, dass** das Rohmethanol in einem thermischen Trennverfahren in Reinmethanol und Wasser aufgetrennt wird, und wobei in dem thermischen Trennverfahren ein kohlenstoffhaltiger Abgasstrom erhalten wird, wobei der kohlenstoffhaltige Abgasstrom als Brennstoff in einer Heizvorrichtung zum Vorheizen des gemäß Schritt (e) erhaltenen Stroms genutzt wird, bevor dieser Strom gemäß Schritt (f) zu Synthesegas umgesetzt wird.

16. Verfahren nach Anspruch 14 oder 15, **dadurch gekennzeichnet, dass** das im thermischen Trennverfahren abgetrennte Wasser als Ausgangsmaterial für den elektrolytisch erzeugten Wasserstoffstrom verwendet wird.

17. Verfahren nach einem der Ansprüche 14 bis 16, **dadurch gekennzeichnet, dass** beim Umsetzen des Synthesegasstroms an dem festen Methanolsynthese-Katalysator zu Rohmethanol ein Restgasstrom erzeugt wird, welcher nicht zu Rohmethanol umgesetztes Synthesegas enthält, und wobei von dem Restgasstrom ein Teil als Spülgasstrom abgetrennt wird, und wobei der Spülgasstrom einer Wasserstoffrückgewinnungsvorrichtung zugeführt wird, wodurch ein nicht elektrolytisch erzeugter Wasserstoffstrom erzeugt wird, und

- der nicht elektrolytisch erzeugte Wasserstoffstrom zumindest teilweise zusätzlich dem schwefelhaltigen Kohlenwasserstoffstrom zugeführt wird, wodurch der mit Wasserstoff angereicherte schwefelhaltige Kohlenwasserstoffstrom erhalten wird, und/oder
- der nicht elektrolytisch erzeugte Wasserstoffstrom zumindest teilweise zusätzlich dem gemäß Schritt (d) erhaltenen Strom zugeführt wird, wodurch der mit Wasserstoff angereicherte, schwefelfreie Kohlenwasserstoff-

strom erhalten wird.

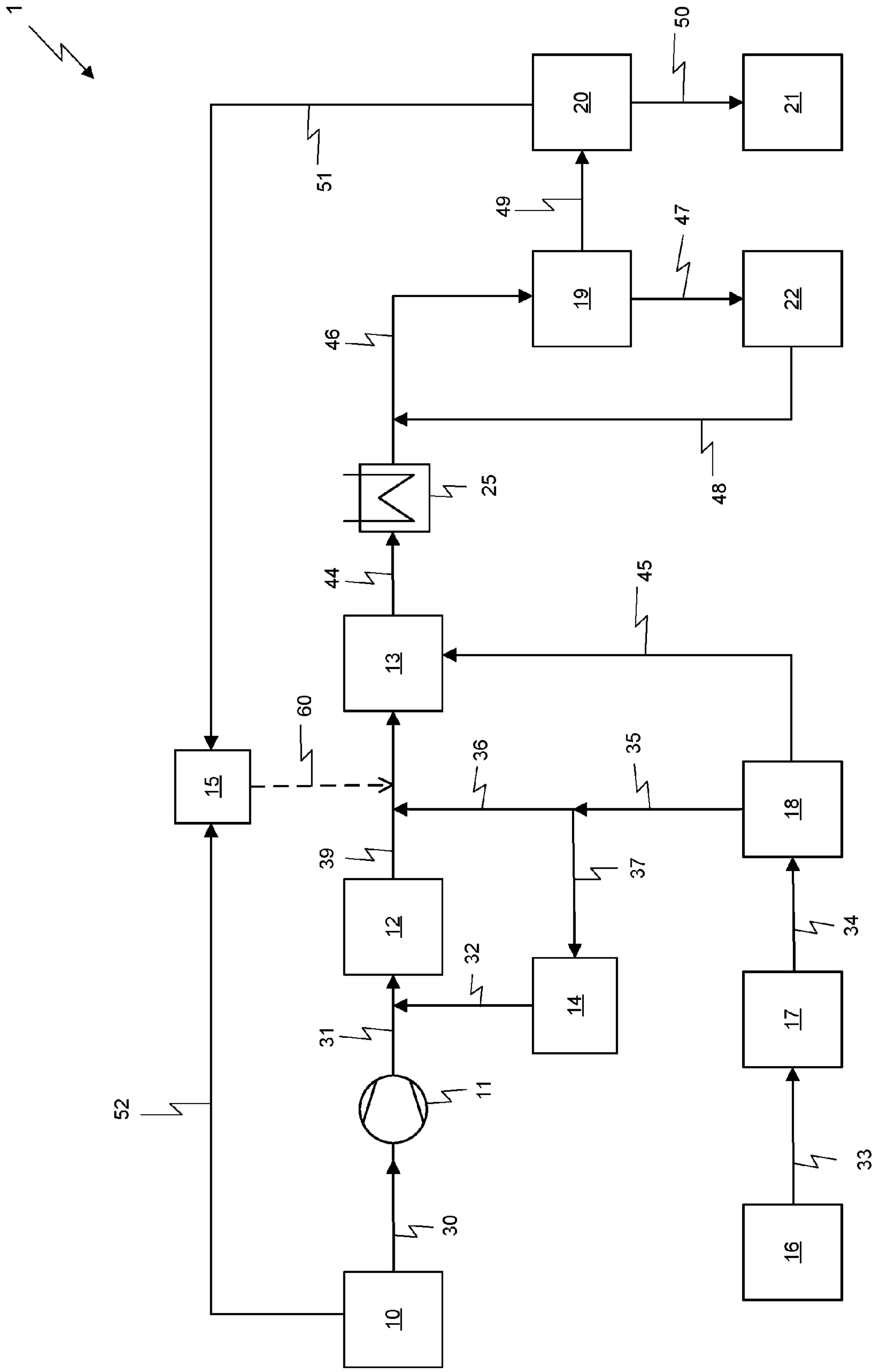
1
10
11
12
13
14
15
16
17
18
19
20
21
22
25
30
31
32
33
34
35
36
37
39
44
45
46
47
48
49
50
51
52
60

Fig. 1

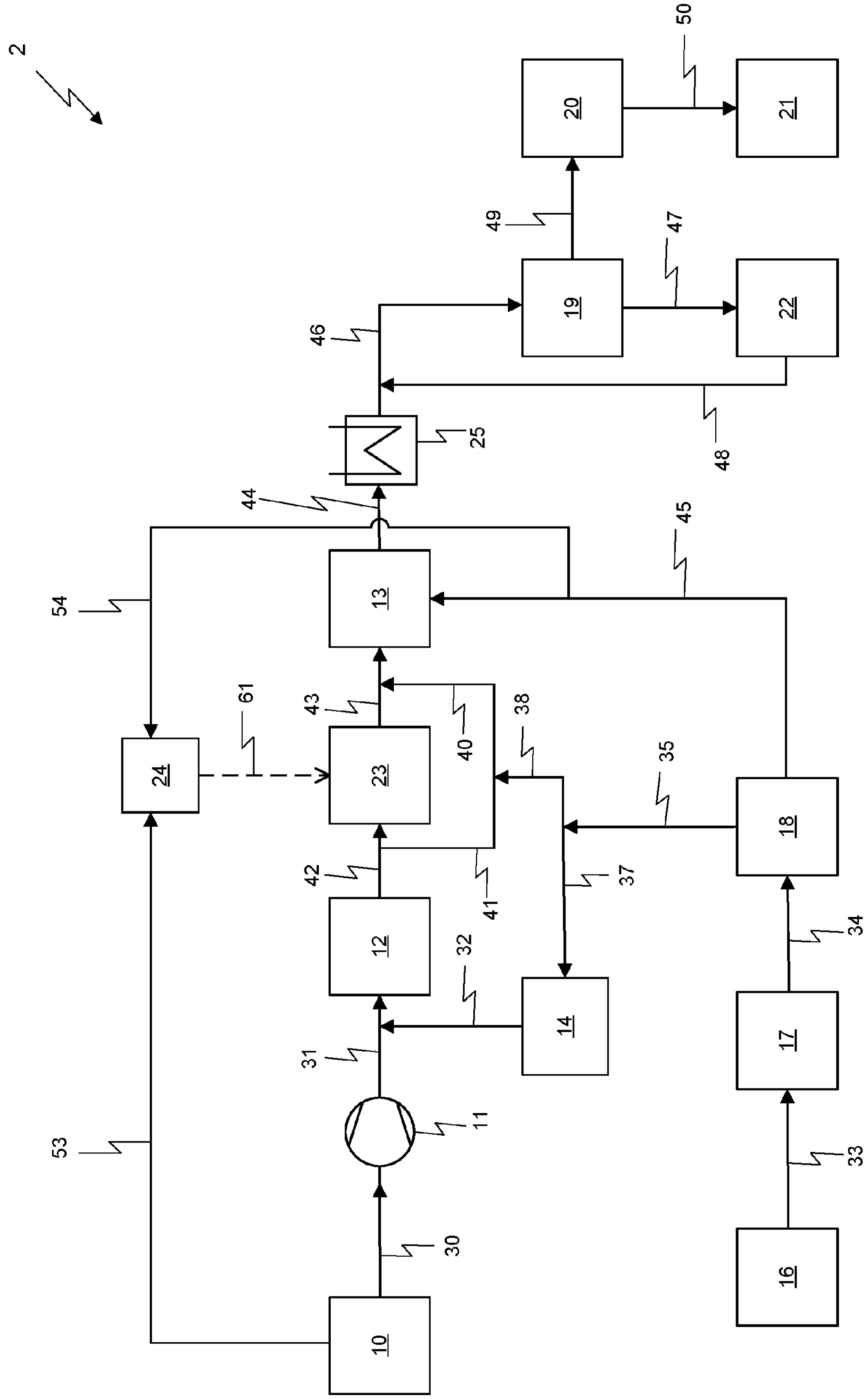
2
10
11
12
13
14
16
17
18
19
20
21
22
23
24
25
30
31
32
33
34
35
37
38
40
41
42
43
44
45
46
47
48
49
50
53
54
61

Fig. 2

Europäisches Patentamt
European Patent Office
Office européen des brevets

# EUROPÄISCHER RECHERCHENBERICHT

Nummer der Anmeldung
EP 23 20 8276

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| E | EP 4 324 815 A1 (AIR LIQUIDE [FR]) 21. Februar 2024 (2024-02-21) * Absätze [0037], [0076] - [0082]; Abbildung 1 * ----- | 1,3,8, 10,14 | INV. C01B3/36 C01B3/38 C07C29/151 C25B1/04 |
| A | WO 2023/187147 A1 (TOPSOE AS [DK]) 5. Oktober 2023 (2023-10-05) * Seite 21, Zeile 17 - Seite 22, Zeile 28; Abbildungen 2,3 * ----- | 1-17 | |
| A | US 2023/061332 A1 (STROFFOLINO IV JOSEPH T [SG]) 2. März 2023 (2023-03-02) * Absätze [0150] - [0156]; Abbildung 2 * ----- | 1-17 | |
| A | WO 2019/122809 A1 (JOHNSON MATTHEY DAVY TECHNOLOGIES LTD [GB]) 27. Juni 2019 (2019-06-27) * Seite 14, Zeile 28 - Seite 16, Zeile 9; Abbildung 1 * ----- | 1-17 | |

RECHERCHIERTE SACHGEBIETE (IPC)

C01B
C07C
C25B

Der vorliegende Recherchenbericht wurde für alle Patentansprüche erstellt

1

| Recherchenort | Abschlußdatum der Recherche | Prüfer |
|---|---|---|
| München | 10. April 2024 | Werner, Håkan |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

# ANHANG ZUM EUROPÄISCHEN RECHERCHENBERICHT ÜBER DIE EUROPÄISCHE PATENTANMELDUNG NR.

EP 23 20 8276

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-04-2024

| Im Recherchenbericht angeführtes Patentdokument | Datum der Veröffentlichung | Mitglied(er) der Patentfamilie | Datum der Veröffentlichung |
|---|---|---|---|
| EP 4324815 A1 | 21-02-2024 | CN 117586098 A | 23-02-2024 |
| | | EP 4324815 A1 | 21-02-2024 |
| | | US 2024059637 A1 | 22-02-2024 |
| WO 2023187147 A1 | 05-10-2023 | KEINE | |
| US 2023061332 A1 | 02-03-2023 | KEINE | |
| WO 2019122809 A1 | 27-06-2019 | AU 2018390366 A1 | 02-07-2020 |
| | | CA 3083847 A1 | 27-06-2019 |
| | | GB 2570043 A | 10-07-2019 |
| | | RU 2020120100 A | 17-12-2021 |
| | | US 2021017023 A1 | 21-01-2021 |
| | | US 2022144654 A1 | 12-05-2022 |
| | | WO 2019122809 A1 | 27-06-2019 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 3658494 B1